(19) Europäisches Patentamt
European Patent Office
Office européen des brevets

(11) Veröffentlichungsnummer: **0 000 377**
**B1**

(12) # EUROPÄISCHE PATENTSCHRIFT

(21) Anmeldenummer: **78100318.1**

(22) Anmeldetag: **06.07.78**

(51) Int. Cl.³: **C 07 D 311/24,**
A 01 N 43/16,
C 07 D 311/92

(54) **Verfahren zur Herstellung von Chromon-Derivaten, neue Chromon-Derivate sowie die Verwendung der neuen Derivate als Pflanzenschutzmittel**

(30) Priorität: **13.07.77 DE 2731566**

(43) Veröffentlichungstag der Anmeldung:
**24.01.79 Patentblatt 79/02**

(45) Bekanntmachung des Hinweises auf die Patenterteilung:
**21.01.81 Patentblatt 81/03**

(84) Benannte Vertragsstaaten:
**BE CH DE FR GB NL**

(56) Entgegenhaltungen:
**JOURNAL OF THE CHEMICAL SOCIETY**
**Perkin Transactions I (22) 1974,**
**Seiten 2570—2574, Benzopyrones,**
**Part XI Some 3-substituted 4-oxo-chromen-2-carboxilic acid derivates**
**by Gwynn P. Ellis and Idris L. Thomas**

(73) Patentinhaber: **BAYER AG**
**Zentralbereich Patente, Marken und Lizenzen**
**D-5090 Leverkusen 1, Bayerwerk (DE)**

(72) Erfinder: **Kabbe, Hans-Joachim, Dr.**
**Walter-Flex-Strasse 16**
**D-5090 Leverkusen 1 (DE)**
**Frohberger, Paul-Ernst, Dr.**
**Willi-Baumeister-Strasse 5**
**D-5090 Leverkusen 1 (DE)**
**Roessler, Peter, Dr.**
**Elster-Strasse 15**
**D-5060 Bergisch-Gladbach 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 000 377 B1

Courier Press, Leamington Spa, England.

# Verfahren zur Herstellung von Chromon-Derivaten, neue Chromon-Derivate sowie die Verwendung der neuen Derivate als Pflanzenschutzmittel

Die Erfindung betrifft ein chemisch eigenartiges Verfahren zur Herstellung von Chromon-Derivaten, sowie Chromon-Derivate und deren Verwendung als Pflanzenschutzmittel.

Es ist bereits bekannt, daß aromatische Aldehyde in Gegenwart von Natronlauge mit o-Hydroxyacetophenonen zu 2-Phenyl-chromanonen reagieren (vgl. Elderfield, "Heterocyclic Compounds", Vol. 2, Seite 347), ferner ist noch bekannt, daß man auch aliphatische Aldehyde mit o-Hydroxyacetophenonen zu den entsprechenden 2-Alkylchromanonen umsetzen kann (vgl. DE—OS 2 535 338). In beiden Fallen reagieren o-Hydroxyacetophenon und Aldehyd im Molverhältnis 1:1. Weiterhin ist noch bekannt, daß man aus o-Acylphenolen und Carbonsäure-Derivaten ebenfalls im Verhältnis 1:1 Chromone herstellen kann [vgl. P. Karrer, "Lehrbuch der Organischen Chemie", 13. Auflage, Seite 584, Georg Thieme Verlag, Stuttgart (1959)]

Als Pflanzenschutzmittel mit fungizider Wirkung ist als Standard-Präparat mit weltweiter Verbreitung Zink-äthylenbis-dithiocarbamidat bekannt [vgl. R. Wegler, "Chemie der Pflanzenschutz- und Schädlingsbekämpfungsmittel", Band 4, Seite 139, Springer-Verlag, Berlin/Heidelberg/New York (1977)]. Bei niedrigen Aufwandmengen ist die Wirkung jedoch nicht immer befriedigend.

Wirkstoffe, die die Metamorphose von Arthropoden hemmen, sind erst seit jüngerer Zeit im Pflanzenschutz von Interesse. Zu nennen ist hier z.B. das 2,2-Dimethyl-6-methoxybenzopyran [vgl. Chem. Eng. News *54,* 19—20 (1976)].

Es wurde gefunden, daß man die Chromon-Derivate der allgemeinen Formel I

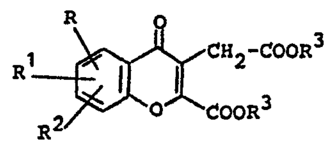

(I),

in welcher

R, $R^1$ und $R^2$ gleich oder verschieden sein können und für Wasserstoff, für Alkyl, Cycloalkyl, Alkenyl und Cycloalkenyl mit jeweils bis zu 8 Kohlenstoffatomen stehen, wobei die Alkyl- oder Cycloalkylreste gegebenenfalls durch Halogen, Cyan, Alkoxy- und Alkoxycarbonyl-Gruppen mit bis zu 4 Kohlenstoffatomen substituiert sein können; ferner für Phenyl, Naphthyl, Phenylalkyl und Naphthylalkyl mit 1 bis 4 Kohlenstoffatomen im Alkylteil stehen, wobei die genannten aromatischen Reste durch Halogen, Cyano, Alkyl-, Alkoxy-, Alkoxycarbonyl- und Alkoxycarbonylalkyl- mit bis zu 4 Kohlenstoffatomen und Di-alkyl-amino-Gruppen mit insgesamt bis zu 6 Kohlenstoffatomen substituiert sein können, ferner für gegebenenfalls durch Halogen oder Cyano substituiertes Alkoxy mit bis zu 4 Kohlenstoffatomen stehen; sodann für Phenoxy, Naphthoxy und Phenylalkoxy mit bis zu 4 Kohlenstoffatomen in Alkylteil stehen; weiterhin noch für Halogen, Cyano- und Hydroxy-Gruppen stehen, und schließlich für Alkoxycarbonyl- mit bis zu 4 und für Dialkylamino-Gruppen mit bis zu 3 Kohlenstoffatomen im Alkylteil stehen, wobei die beiden Alkylreste auch mit dem Aminostickstoffatom zu einem heterocyclischen Ring geschlossen sein können, und weiterhin zwei Reste

R und $R^1$ zusammen mit 2 Kohlenstoffatomen des Benzolringsystems einen carbocyclischen oder heterocyclischen 5- oder 6-Ring bilden können, und

$R^3$ für Wasserstoff und für Alkyl mit bis zu 4 Kohlenstoffen steht, wobei letzteres durch Halogen und Methoxy-Gruppen substituiert sein kann, ferner für Allyl, Cyclopentyl und Cyclohexyl, für Benzyl, Chlorbenzyl und für Phenyläthyl steht,

in einfacher Weise erhält, wenn man o-Hydroxyacetophenone der allgemeinen Formel II

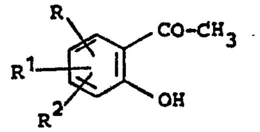

(II)

in welcher

R, $R^1$ und $R^2$ die oben angegebene Bedeutung haben, mit Glyoxylsäure-Derivaten der allgemeinen Formel III

$$OCH—COOZ \qquad (III)$$

in welcher

Z für ein- oder zweiwertige Kationen von Alkali- und Erdalkalimetallen, sowie für das Ammonium-, Mono-, Di- und Trialkylammonium-Kation stehen, wobei die Alkylreste jeweils bis zu 2 Kohlenstoffatome enthalten,

in Gegenwart von basischen Verbindungen im Temperaturbereich zwischen −30 und +150°C umsetzt und noch gegebenenfalls die dabei nach dem Ansäuern erhaltenen Carbonsäuren in an sich bekannter Weise in die entsprechenden Ester überführt.

Weiterhin wurden als neue Verbindungen die Chromon-Derivate der allgemeinen Formel Ia

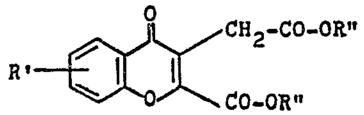

(Ia)

in welcher

R′ für Wasserstoff, Chlor oder Methoxy steht, und

R″ für Wasserstoff oder Methyl steht, gefunden.

Die neuen Verbindungen der Formel Ia besitzen fungizide Eigenschaften; ferner hemmen sie die Entwicklung von Arthropoden. Sie sind daher als Pflanzenschutzmittel von Interesse.

Es ist als ausgesprochen überraschend zu bezeichnen, daß die o-Hydroxyacetophenone der Formel II mit den Glyoxylsäure-Derivaten der Formel III in einer einfachen und überschaubaren Reaktion die Chromon-Derivate der Formel I ergeben und keine Chromanon-Verbindungen entstehen. Das Auffinden der neuen Reaktion stellt eine Bereicherung der Technik dar. Von praktischem Interesse ist, daß die neuen Verbindungen als Pflanzenschutzmittel verwendet werden können.

Setzt man o-Hydroxyacetophenon mit dem Natriumsalz der Glyoxylsäure in Gegenwert von Pyrrolidin um und verestert die dabei erhaltene Dicarbonsäure anschließend mit Methanol, so kann der Reaktionsablauf durch das folgende Reaktionsschema wiedergegeben werden:

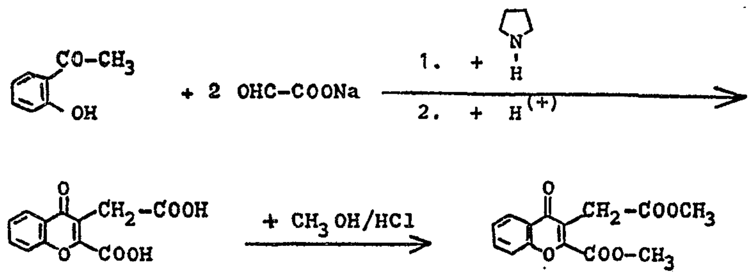


In der Formel II stehen R, $R^1$ und $R^2$ vorzugsweise für Wasserstoff und für gegebenenfalls substituiertes, geradkettiges oder verzweigtes Alkyl mit bis zu 8, bevorzugt bis zu 2 Kohlenstoffatomen, ferner für geradkettiges oder verzweigtes Alkenyl mit einer oder mehreren Doppelbindungen und bis zu 8 Kohlenstoffatomen, vorzugsweise bis zu 3 Kohlenstoffatomen und einer Doppelbindung. Als Alkyl- bzw. Alkenyl-Reste seien beispielsweise genannt: Methyl, Äthyl, Propyl, Isopropyl, Butyl, Isobutyl, tert.-Butyl, Hexyl, Buten-(3)-yl und 4-Methyl-penten-(3)-yl.

R, $R^1$ und $R^2$ stehen weiterhin für gegebenenfalls substituierte Cycloalkyl- und Cycloalkenyl-Reste mit 3 bis 8, bevorzugt mit 4 bis 6 Kohlenstoffatomen, wie beispielsweise Cyclobutyl und insbesondere Cyclopentyl und Cyclohexyl.

R, $R^1$ und $R^2$ stehen weiterhin für Phenyl und Naphthyl, bevorzugt Phenyl.

Als gegebenenfalls substituierte Phenylalkyl- und Naphthylalkyl-Reste seien beispielhaft genannt: Benzyl, Phenyläthyl, Phenylpropyl, Phenylbutyl, Naphthylmethyl und Naphthyläthyl, bevorzugt Benzyl..

Als Alkoxygruppen seien solche mit bis zu 4 Kohlenstoffatomen, wie Methoxy, Äthoxy, Propoxy, Isopropoxy, Butoxy, Isobutoxy und tert.-Butoxy, gennant.

Als bevorzugte Phenylalkoxygruppen seien Benzyloxy, Phenyläthoxy, Phenylpropoxy, Phenylisopropoxy, Phenylbutoxy, Phenylisobutoxy und Phenyl-tert.-butoxy, genannt.

Als bevorzugte Alkoxycarbonylgruppen seien Methoxycarbonyl, Äthoxycarbonyl, Propoxycarbonyl, Isopropoxycarbonyl, genannt.

Als bevorzugte Dialkylaminogruppen seien Dimethylamino, Diäthylamino, und Diisopropylamino, genannt. Es ist auch möglich, daß die beiden Alkylreste der Dialkylaminogruppe zu einem Ring geschlossen sind, wie beispielsweise Pyrrolidinyl, Piperidinyl.

Als Halogene seien Fluor, Chlor, Brom und Jod, bevorzugt Chlor und Brom genannt.

Die Reste R und $R^1$ können mit den beiden Kohlenstoffatomen des Benzolrings, an dem sie sitzen, einen carbocyclischen oder heterocyclischen fünf- oder sechsgliedrigen Ring bilden, wie z.B. einen Cyclopenten-, Cyclohexen-, Benzol-, Furan-, Dihydrofuran-, Thiophen-, Dihydrothiophen-, Pyran-, Dihydropyran-, Pyridin- oder Dioxolen-Ring.

Als Substituenten der Alkyl-, Cycloalkyl-, Aryl-, Aralkyl-, Alkoxy-, Aralkoxy-, Alkoxycarbonyl- und Dialkylamino-Gruppen der Reste R bis $R^2$ kommen Substituenten in Frage, die unter den Reaktionsbedingungen nicht verändert werden. Beispielsweise seien die Halogene, wie Fluor, Chlor, Brom und

Jod und die Cyangruppe genannt, ferner die Alkoxy- und Alkoxycarbonyl-Gruppe mit bis zu 4, und — sofern es sich um Substituenten an Ringsystemen handelt — noch die Alkyl- und die Alkoxycarbonylalkyl- Gruppe mit bis zu 4 Kohlenstoffatomen. Ferner sind als weitere Zweitsubstituenten noch die Dialkylamino-Gruppe mit insgesamt bis zu 6, vorzugsweise bis zu 2 Kohlenstoffatomen zu nennen.

Die o-Hydroxyacetophenone der Formel II, die für das erfindungsgemäße Verfahren Verwendung finden können, sind bekannt (vgl. Beilstein, Handbuch der Organischen Chemie, *H8*, Seite 85 ff.). Beispielsweise seien genannt:

o-Hydroxyacetophenon
3-Chlor-2-hydroxyacetophenon
5-Chlor-2-hydroxyacetophenon
3,5-Dichlor-2-hydroxyacetophenon
3-Methyl-5-chlor-2-hydroxyacetophenon
2,4-Dihydroxyacetophenon
2,5-Dihydroxyacetophenon
2,6-Dihydroxyacetophenon
2,3-Dihydroxyacetophenon
2,4,6-Trihydroxyacetophenon
4-Pentyl-2,6-dihydroxyacetophenon
4-Heptyl-2,6-dihydroxyacetophenon
4-(1',1'-Dimethylpentyl)-2,6-dihydroxyacetophenon
3,4-Dimethoxy-6-methyl-2-hydroxyacetophenon
3,4,6-Trimethyl-2-hydroxyacetophenon
3-Methoxy-2-hydroxyacetophenon
4-Methoxy-2-hydroxyacetophenon
5-Methoxy-2-hydroxyacetophenon
6-Methoxy-2-hydroxyacetophenon
4-Benzyloxy-2-hydroxyacetophenon
5-Benzyloxy-2-hydroxyacetophenon
4-Phenoxy-2-hydroxyacetophenon
4-Cyclohexyl-2-hydroxyacetophenon
5-Phenyl-2-hydroxyacetophenon
3-β-Phenyläthyl-2-hydroxyacetophenon
5-δ-Phenylbutyl-2-hydroxyacetophenon
3,5-Dibrom-2-hydroxyacetophenon
4-Äthoxy-2-hydroxyacetophenon
5-Äthoxycarbonyl-äthoxy-2-hydroxyacetophenon
4-Methoxycarbonyl-methoxy-2-hydroxyacetophenon
4-Carboxymethyl-2-hydroxyacetophenon
5-Nitro-2-hydroxyacetophenon
3-Cyan-2-hydroxyacetophenon
4-Trifluormethyl-2-hydroxyacetophenon
5-Trifluormethyl-2-hydroxyacetophenon
3-Trifluormethyl-2-hydroxyacetophenon
3-Methoxycarbonyl-2-hydroxyacetophenon
5-Carboxy-2-hydroxyacetophenon
5-Dimethylamino-2-hydroxyacetophenon
4-N-Piperidinyl-2-hydroxyacetophenon
3-Phenoxy-2-hydroxyacetophenon
4-p-Chlorphenoxy-2-hydroxyacetophenon
5-p-Tolyl-2-hydroxyacetophenon
1-Hydroxy-2-acetylnaphthalin
2-Hydroxy-1-acetylnaphthalin

Weiterhin werden als Ausgangsverbindungen noch die Glyoxylsäure-Derivate der Formel III benötigt. In der Formel III steht Z für ein- oder zweiwertige Kationen von Alkali- und Erdalkali-Metallen, sowie für das Ammonium-Kation und die Mono-, Di, und Trialkylammonium-Kationen. In den drei letztgenannten Fällen enthalten die Alkylreste bis zu 2 Kohlenstoffatome.

Die Synthese wird in Gegenwart von basischen Verbindungen durchgeführt. Als solche kommen bevorzugt sekundäre Amine in Frage, insbesondere cyclische Amine wie Pyrrolidin, Piperidin, N-Methylpiperazin und, Morpholin, aber auch offenkettige Amine, wie Dimethylamin und Diäthylamin. Die genannten Verbindungen sind allgemein bekannt.

Das erfindungsgemäße Verfahren kann mit oder ohne Lösungsmittel durchgeführt werden. Zur Durchführung des Verfahrens kommen alle Lösungsmittel in Betracht, die gegenüber den Ausgangskomponenten und dem Endprodukt inert sind. Als Lösungsmittel seien beispielsweise gennant: Aliphatische oder aromatische Kohlenwasserstoffe, wie Petroläther, Benzol, Toluol oder Xylol, aliphatische oder aromatische Halogenkohlenwasserstoffe, wie Tetrachlorkohlenstoff, Chlorbenzol oder Dichlorbenzol, Äther, wie Diäthyläther, Tetrahydrofuran, Dioxan oder Glykoldimethyläther, sodann Amide, wie Dimethylformamid, Dimethylacetamid und N-Methylpyrrolidon, ferner Ester wie Essigsäureäthylester, Nitrile, wie Acetonitril, Propionnitril, und Alkohole, wie Methanol, Äthanol und Glykolmonomethyläther, und schließlich Wasser.

Das erfindungsgemäße Verfahren wird bei einer Temperatur von −30 bis +150°C, bevorzugt von 20 bis 80°C, durchgeführt.

Zur Durchführung des erfindungsgemäßen Verfahrens werden im allgemeinen die o-Hydroxyacetophenone (II) und das Salz der Glyoxylsäure (III) in stöchiometrischen Mengen, also im Verhältnis 1:2, eingesetzt. Für die Durchführung des erfindungsgemäßen Verfahrens ist es jedoch ohne Bedeutung, wenn ein kleiner Überschuß einer Komponente, insbesondere des Glyoxylsäuresalzes, eingesetzt wird (z.B. bis zum Molverhältnis 1:2,5). Die Menge des eingesetzten Amins ist nicht kritisch. Im allgemeinen verwendet man 0,05 bis 1,5, bevorzugt 0,1 bis 1 Mol des Amins, bezogen auf 1 Mol der o-Hydroxycarbonyl-Verbindung. Falls die o-Hydroxyacetophenone durch sauer reagierende Gruppen wie z.B. Carboxygruppen, substituiert sind, kann es zweckmäßig sein, durch einen Überschuß des Amins die sauren Gruppen zu neutralisieren.

Im allgemeinen wird das erfindungsgemäße Verfahren wie folgt ausgeführt:

Die Ausgangsverbindungen werden bei der gewählten Reaktionstemperatur, gegebenenfalls in einem Lösungsmittel, gelöst und mit dem Amin versetzt. Durch die exotherme Reaktion steigt die Reaktionstemperatur im allgemeinen an, so daß eine weitere Erwärmung nicht notwendig ist. Man läßt dann das Reaktionsgemisch bis zur Beendigung der Reaktion ohne weitere Erwärmung stehen, kann jedoch auch die Reaktionszeit durch äußeres Erwärmen abkürzen. Nach Beendigung der Reaktion isoliert man die Chromon-Derivate, indem man die entstandenen Salze in Wasser löst und diese Lösungen ansäuert, beispielsweise mit Mineralsäuren wie Salz-, Schwefel- oder Phosphorsäure. Man erhalt dann die Dicarbonsäure-Verbindungen der Formel I, wobei $R^3$ für Wasserstoff steht.

Die Veresterung der so erhaltenen Dicarbonsäuren kann beispielsweise in üblicher Weise dadurch erfolgen, daß man die Dicarbonsäure in der 4- bis 100-, vorzugsweise 10- bis 50-fachen molaren Menge Alkohol der Formel $R^3$—OH verrührt, mit einer Säure wie konz. Schwefelsäure versetzt oder mit Chlorwasserstoff-Gas sättigt und einige Stunden auf Temperaturen von 60 bis 120°C erwärmt. Man kann das Gemisch der Dicarbonsäure mit dem Alkohol auch mit der mindestens doppelten molaren Menge, bezogen auf die Dicarbonsäure, eines Dehydratisierungsmittels, wie anorganischen Säurehalogeniden, z.B. Thionylchlorid oder Phosphoroxychlorid, versetzen. Schließlich kann man auch Salze der Dicarbonsäuren mit mindestens der doppelten molaren Menge eines Alkylhalogenids in einem Lösungsmittel, wie Dimethylsulfoxid oder Dimethylformamid, zur Reaktion bringen.

Als neue Chromondicarbonsäuren und deren Ester seien beispielweise genannt:

2-Carboxy-3-carboxymethyl-6-chlor-chromon
2-Carboxy-3-carboxymethyl-7-chlor-chromon
2-Carboxy-3-carboxymethyl-8-chlor-chromon
2-Carboxy-3-carboxymethyl-6,8-dichlor-chromon
2-Carboxy-3-carboxymethyl-6-methyl-chromon
2-Carboxy-3-carboxymethyl-7-methyl-chromon
2-Carboxy-3-carboxymethyl-6-isobutyl-chromon
2-Carboxy-3-carboxymethyl-7-äthyl-chromon
2-Carboxy-3-carboxymethyl-6-sek.-butyl-chromon
2-Carboxy-3-carboxymethyl-6-benzyl-chromon
2-Carboxy-3-carboxymethyl-7-phenyl-chromon
2-Carboxy-3-carboxymethyl-5-methoxy-chromon
2-Carboxy-3-carboxymethyl-6-methoxy-chromon
2-Carboxy-3-carboxymethyl-7-methoxy-chromon
2-Carboxy-3-carboxymethyl-8-methoxy-chromon
2-Carboxy-3-carboxymethyl-6-chlor-8-methyl-chromon
2-Carboxy-3-carboxymethyl-6-pyrrolidinyl-chromon
2-Carboxy-3-carboxymethyl-6-dimethylamino-chromon
2-Carboxy-3-carboxymethyl-7,8-dimethoxy-chromon
2-Carboxy-3-carboxymethyl-6,7-dimethoxy-chromon
2-Carboxy-3-carboxymethyl-6-methoxycarbonyl-chromon
2-Carboxy-3-carboxymethyl-8-carboxy-chromon
2-Carboxy-3-carboxymethyl-7-cyan-chromon
2-Carboxy-3-carboxymethyl-6-cyan-chromon
2-Carboxy-3-carboxymethyl-5,6,7-trimethyl-chromon
2-Carboxy-3-carboxymethyl-5,6-benzo-chromon
2-Carboxy-3-carboxymethyl-5,6-trimethylen-chromon

2-Carboxy-3-carboxymethyl-5-methyl-6,8-dichlor-chromon
2-Carbomethoxy-3-carbomethoxymethyl-6-chlor-chromon
2-Carbomethoxy-3-carbomethoxymethyl-7-chlor-chromon
2-Carbomethoxy-3-carbomethoxymethyl-6,8-dichlor-chromon
2-Carbomethoxy-3-carbomethoxymethyl-6-methyl-chromon
2-Carbomethoxy-3-carbomethoxymethyl-7-methyl-chromon
2-Carbomethoxy-3-carbomethoxymethyl-6-isobutyl-chromon
2-Carbomethoxy-3-carbomethoxymethyl-6-cyclopentyl-chromon
2-Carbomethoxy-3-carbomethoxymethyl-7-phenyl-chromon
2-Carbomethoxy-3-carbomethoxymethyl-5-methoxy-chromon
2-Carbomethoxy-3-carbomethoxymethyl-6-methoxy-chromon
2-Carbomethoxy-3-carbomethoxymethyl-7-methoxy-chromon
2-Carbomethoxy-3-carbomethoxymethyl-8-methoxy-chromon
2-Carbomethoxy-3-carbomethoxymethyl-6-chlor-8-methyl-chromon
2-Carbomethoxy-3-carbomethoxymethyl-7,8-dimethoxy-chromon
2-Carbomethoxy-3-carbomethoxymethyl-6,7-dimethoxy-chromon
2-Carbomethoxy-3-carbomethoxymethyl-8-carboxy-chromon
2-Carbomethoxy-3-carbomethoxymethyl-6-cyan-chromon
2-Carbomethoxy-3-carbomethoxymethyl-5,6-benzo-chromon
2-Carbomethoxy-3-carbomethoxymethyl-5-methyl-6,8-dichlor-chromon
2-Carbo-propoxy-3-carbo-propoxymethylchromon
2-Carbo-isopropoxy-3-carbo-isopropoxymethylchromon
2-Carbo-butoxy-3-carbo-butoxymethylchromon
2-Carbo-sek.-butoxy-3-carbo-sek. butoxymethylchromon
2-Carbo-iso-butoxy-3-carbo-iso-butoxymethylchromon
2-Carbo-tert.-butoxy-3-carbo-tert.-butoxymethylchromon
2-Carbo-benzyloxy-3-carbo-benzyloxymethylchromon
2-Carbo-phenäthoxy-3-carbo-phenäthoxymethylchromon
2-Carbo-cyclopentyloxy-3-carbo-cyclopentyloxymethylchromon
2-Carbo-cyclohexyloxy-3-carbo-cyclohexyloxymethylchromon
2-Carbo-allyloxy-3-carbo-allyloxymethylchromon
2-Carbo-p-chlorbenzyloxy-3-carbo-p-chlorbenzyloxymethylchromon
2-Carbo-2′-methoxyäthoxy-3-carbo-2′-methoxyäthoxymethylchromon
2-Carbo-2′-bromäthoxy-3-carbo-2′-bromäthoxymethylchromon

Die erfindungsgemäßen Wirkstoffe weisen eine gute fungitoxische Wirkung auf. Sie schädigen Kulturpflanzen in den zur Bekämpfung von Pilzen notwendigen Konzentrationen nicht. Aus diesen Gründen sind sie für den Gebrauch als Pflanzenschutzmittel zur Bekämpfung von Pilzen geeignet. Fungitoxische Mittel im Pflanzenschutz werden eingesetzt zur Bekämpfung von Plasmodiophoromycetes, Oomycetes, Chytridiomycetes, Zygomycetes, Ascomycetes, Bsidiomycetes, Deuteromycetes.

Die erfindungsgemäßen Wirkstoffe können angewandt werden gegen parasitäre Pilze und Bakterien, die oberirdische Pflanzenteile befallen oder die Pflanzen vom Boden her angreifen, sowie gegen samenübertragbare Krankheitserreger.

Die gute Pflanzenverträglichkeit erlaubt eine Anwendung gegen pilzliche Pflanzenkrankheiten durch Behandlung der stehenden Kulturpflanze oder einzelner Teile von ihr oder des Saatgutes oder auch des Kulturbodens. Die Wirkstoffe sind besonders wirksam gegen Getreidemehltau.

Die erfindungsgemäßen Wirkstoffe können in die üblichen Formulierungen übergeführt werden, wie Lösungen, Emulsionen, Suspensionen, Pulver, Pasten und Granulate. Diese werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Strecktmittel können z.B. auch organische Lösungsmittel als Hilfslösungsmittel verwendet werden. Als flüssige Lösungsmittel kommen im wesentlichen in Frage: Aromaten, wie Xylol, Toluol, Benzol oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylenketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Dichlordifluormethan oder Trichlorfluormethan; als feste Trägerstoffe: natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als Emulgiermittel; nichtionogene und anionische Emulgatoren, wie Polyoxyäthylen-Fettsäure-Ester, Polyoxyäthylen-Fettalkohol-Äther, z.B. Alkylarylpolyglykoläther, Alkylsulfonate, Alkylsulfate, Arylsulfonate sowie Eiweißhydrolysate; als Dispergiermittel: z.B. Lignin-Sulfitablaugen und Methylcellulose.

Die Formulierungen enthalten im allgemeinen zwischen 0,1 und 95 Gewichtsprozent Wirkstoff, vorzugsweise zwischen 0,5 und 90%.

Die Wirkstoffe können als solche, in Form ihrer Formulierungen oder der daraus bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Spritzen, Sprühen, Nebeln, Streuen, Stäuben, Gießen, Trockenbeizen, Schlämmbeizen (Slurrybeizen), Feuchtbeizen und Naßbeizen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 un 10 Gew.-% liegen.

Bei der Beizung kommen im allgemeinen je Kilogramm Saatgut Wirkstoffmengen von 10 mg bis 10 g, vorzugsweise 100 mg bis 3 g zur Anwendung. Bei der Boden behandlung, die ganzflächig, streifenförmig oder punktförmig durchgeführt werden kann, werden am Ort der erwarteten Wirkung Wirkstoffkonzentrationen von 1 bis 1000 g Wirkstoff je $m^3$ Boden benötigt, vorzugsweise 10 bis 200 g pro $m^3$.

Wie schon erwähnt, hemmen die erfindungsgemäßen Verbindungen die Entwicklung von Gliederfüßlern (Arthrophoden).

Durch den im folgenden angegebenen Versuche wird die arthropodenmetamorphosehemmende Wirkung der erfindungsgemäßen Verbindungen gezeigt, ohne eine Beschränkung hinsichtlich der Wirkungsbreite dieser Verbindungen vornehmen zu wollen. Dabei werden während der gesamten angegebenen Entwicklung der Testtiere die morphologischen Veränderungen, wie zur Hälfte verpuppte Tiere, unvollständig geschlüpfte Larven oder Raupen, defekte Flügel, puppale Kutikula bei Imagines sowie das Absterben bewertet. Die Summe der morphologischen Mißbildungen und der Abtötung während der Entwicklung werden bonitiert.

Beispiel A

Entwicklungshemmende Wirkung/Fraßtest

| | | |
|---|---|---|
| Testtiere: | Plutella maculipennis | (Raupen im 4. Entwicklungsstadium) 20 Stück |
| | Phaedon cochleariae | (Larven im 4. Entwicklungsstadium) 20 Stück |
| Futterpflanzen: | Kohlpflanzen (Brasica oleracea) | |
| Lösungsmittel: | 4 Gew.-Teile Aceton | |
| Emulgator: | 1 Gew.-Teil Alkylarylpolyglykoläther | |

Zur Herstellung einer zweckmäßigen Wirkstoffzubereitung vermischt man 2 Gewichtsteile Wirkstoff mit der angegebenen Menge Lösungsmittel, Emulgator und soviel Wasser, daß eine 1%ige Mischung entsteht, die mit Wasser auf die gewünschte Konzentration verdünnt wird.

Die Testtiere werden mit Blättern der Futterpflanzen, die mit einem gleichmäßigen Spritzbelag der Wirkstoffmischung der gewählten Konzentration versehen sind, so daß eine Wirkstoffmenge in ppm ("parts pro million") auf den Blättern erhalten wird, bis zur Entwicklung der Imago gefüttert.

Zur Kontrolle werden nur mit Lösungsmittel und Emulgator der angegebenen Konzentration versehene Blätter verfüttert.

Die Versuchsauswertung ergab, daß insbesondere die folgenden erfindungsgemäßen Verbindungen einem bekannten Vergleichspräparat (2,2-Dimethyl-6-methoxy-benzopyran) überlegen sind:

Verbindungen gemäß Herstellungsbeispielen 3, 5 und 10.

Beispiel B

Sproßbehandlungs-Test/Getreidemehltau/protektiv
(blattzerstörende Mykose)

Zur Herstellung einer zweckmäßigen Wirkstoffzubereitung nimmt man 0,25 Gewichsteile Wirkstoff in 25 Gewichtsteilen Dimethylformamid und 0,06 Gewichtsteilen Emulgator (Alkylarylpolyglykoläther) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperaturen von 21 bis 22°C und einer Luftfeuchtigkeit von 80 bis 90% wertet man den Besatz der Pflanzen mit Mehltaupusteln aus. Der Befallsgrad wird in Prozenten des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist umso wirksamer, je geringer der Mehltaubefall ist.

Wirkstoffe, Wirkstoffkonzentrationen in der Spritzbrühe und Befallsgrade gehen aus der nachfolgenden Tabelle hervor:

Tabelle
Sproßbehandlungs-Test/Getreidemehltau/protektiv

| Wirkstoffe | Wirkstoffkonzentration in der Spritzbrühe in Gew.-% | Befall in % der unbehandelten Kontrolle |
|---|---|---|
| unbehandelt | | 100,0 |
| 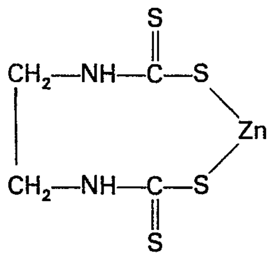 (bekannt) | 0,025 | 100,0 |
| 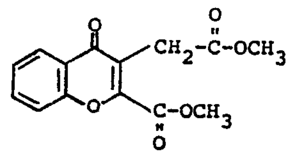 | 0,025 | 0,0 |
| 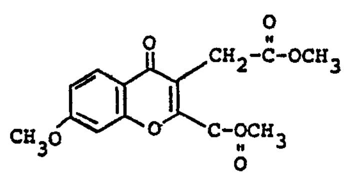 | 0,025 | 20,0 |
| 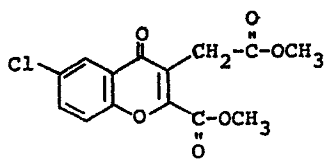 | 0,025 | 38,5 |

Beispiel 1

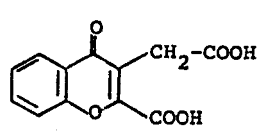

75 g (0,55 m) o-Hydroxyacetophenon und 350 ml Wasser/Eis werden mit 60 ml Pyrrolidin verrührt. In die klare Lösung gibt man 250 g Natriumglyoxylat (Hydrat, ca. 50%ig; 1,1 Mol) und rührt ohne Kühlung nach. Nach einigen Stunden ist das Salz fast klar gelöst; später scheidet sich ein Teil des Endproduktes als Di-Natrium-Salz ab. Nach 5 Tagen versetzt man mit ca. 600 ml Wasser, rührt nach, bis eine fast klare Lösung entsteht und versetzt das Filtrat mit konz. Salzsäure, bis ein pH-Wert von 1 erreicht ist. Der ausfallende Niederschlag wird nach 1 Tag abgesaugt und getrocknet. Ausbeute: 125 g 2-Carboxy-3-carboxymethyl-chromon vom Fp. 240—242°C, das sind 92% der Theorie.

Beispiel 1 a

Man arbeitet wie in Beispiel 1 angegeben, erwärmt aber nach dem Zusammengeben der Ausgangstoffe währen 30 Minuten auf 95°C. Die Aufarbeitung ergibt 112 g 2-Carboxy-3-carboxymethyl-chromon, das sind 82% der Theorie.

Beispiel 2

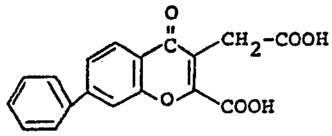

63 g 4-Phenyl-2-hydroxyacetophenon, 700 ml isopropanol und 110 g einer 50%igen Glyoxylsäure-Lösung in Wasser werden verrührt und bei einer Temperatur unterhalb von 0°C mit 63 g Pyrrolidin versetzt. Nach 6 Tagen wird die entstandene Lösung mit 1 Liter Wasser verdünnt und angesäuert. Ausbeute: 22 g 2-Carboxy-3-carboxymethyl-7-phenyl-chromon vom Fp. 219—221°C.

Beispiel 3
(Veresterungs-Reaktion)

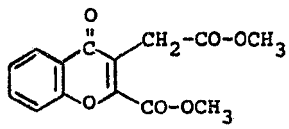

Man verrührt 220 g 2-Carboxy-3-carboxymethyl-chromon (Herstellung gemäß Beispiel 1) in 2 Liter Methanol, leitet Chlorwasserstoff-Gas bis zur Sättigung ein und erwärmt gleichzeitig, bis die Rückflußtemperatur erreicht ist. Nach 8 Stunden läßt man abkühlen und saugt ab. Man erhält 196 g 2-Methoxycarbonyl-3-methoxycarbonylmethyl-chromon vom Fp 105°C.

Beispiel 3 a
(Veresterungs-Reaktion, vgl. Beispiel 3)

Man verrührt 15 g 2-Carboxy-3-carboxymethyl-chromon in 60 ml Methanol und versetzt in ca. 10 Minuten mit 15 ml Thionylchlorid, wobei man die Temperatur unter 35°C hält. Anschließend heizt man während 2 Stunden auf 60°C, läßt abkühlen und saugt ab. Man erhält 13,5 g des gleichen Produktes wie in Beispiel 3 angegeben.

Beispiel 4
(Veresterungs-Reaktion)

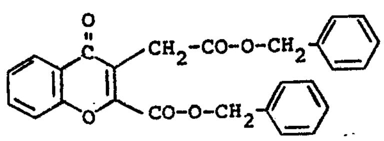

Man löst 25 g 2-Carboxy-3-carboxymethyl-chromon in 30 ml Dimethylsulfoxid und versetzt nacheinander mit 30 ml Triäthylamin und 30 g Benzylchlorid. Nach 24 Stunden gießt man auf Eiswasser, schüttelt mit Chloroform aus und wäscht die Chloroform-Schicht mit 100 ml 2n-Natronlauge und dann mit Wasser. Nach dem Trocknen und Einengen erhält man einen Rückstand, der aus Äther umkristallisiert wird. Die Ausbeute beträgt 17 g 2-Benzyloxycarbonyl-3-benzyloxycarbonylmethyl-chromon vom Fp. 82—84°C.

In ähnlicher Weise wie in den Beispielen 1 bis 4 beschrieben, lassen sich die folgenden Verbindungen der allgemeinen Formel I

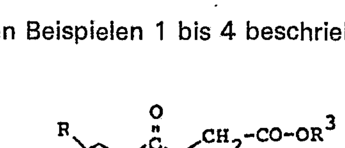

(I)

herstellen.

| Beispiel Nr. | R | $R^1$ | $R^2$ | $R^3$ | Ausbeute % d. Th. | Fp (°C) |
|---|---|---|---|---|---|---|
| 5 | 6-Cl | H | H | H | 77 | 219—221 |
| 6 | 7-Cl | H | H | H | 84 | 150—152 |
| 7 | 6-Cl | 8-Cl | H | H | 66 | 230—232 |
| 8 | 6-Cl | 8-$CH_3$ | H | H | 74 | 170—172 |
| 9 | 6-$CH_3O$ | H | H | H | 77 | 225—227 |
| 10 | 7-$CH_3O$ | H | H | H | 61 | 239—241 |
| 11 | 7-$CH_3O$ | 8-$CH_3O$ | H | H | 80 | 230—232 |
| 12 | 6-$CH_3$ | H | H | H | 83 | 204—206 |
| 13 | 7-$CH_3$ | H | H | H | 64 | 147—148 |
| 14 | 5,6—CH=CH—CH=CH | | H | H | 51 | Zers. über 150 |
| 15 | H | H | H | $C_2H_5$ | 60 | 63—65 (bekannt) |
| 16 | 7-$CH_3O$ | H | H | $CH_3$ | 87 | 118—120 |
| 17 | 6-Cl | 8-Cl | H | $CH_3$ | 90 | 139—141 |
| 18 | 6-Cl | H | H | $CH_3$ | 88 | 169—171 |
| 19 | 7-Cl | H | H | $CH_3$ | 87 | 147—148 |
| 20 | 7-Cl | H | H | $CH_3$ | 70 | 123—125 |
| 21 | 6-Cl | 8-$CH_3$ | H | $CH_3$ | 65 | 138—141 |
| 22 | 7-$CH_3O$ | 8-$CH_3$ | H | $CH_3$ | 63 | 163—165 |
| 23 | 6-$CH_3$ | H | H | $CH_3$ | 51 | 155—157 |

**Patentansprüche**

1. Verfahren zur Herstellung von Chromon-Derivaten der allgemeinen Formel I

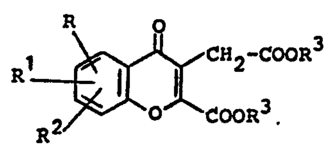

(I)

in welcher

R, $R^1$ und $R^2$ gleich oder verschieden sein können und für Wasserstoff, für Alkyl, Cycloalkyl, Alkenyl und Cycloalkenyl mit jeweils bis zu 8 Kohlenstoffatomen stehen, wobei die Alkyl- oder Cycloalkylreste gegebenenfalls durch Halogen, Cyan, Alkoxy- und Alkoxycarbonyl-Gruppen mit bis zu 4 Kohlenstoffatomen substituiert sein können; ferner für Phenyl, Naphthyl, Phenylalkyl und Naphthylalkyl mit 1 bis 4 Kohlenstoffatomen im Alkylteil stehen, wobei die genannten aromatischen Reste durch Halogen, Cyano, Alkyl-, Alkoxy-, Alkoxycarbonyl- und Alkoxycarbonylalkyl- mit bis zu 4 Kohlenstoffatomen und Di-alkyl-amino-Gruppen mit insgesamt bis zu 6 Kohlenstoffatomen substituiert sein können, ferner für gegebenenfalls durch Halogen oder Cyano substituiertes Alkoxy mit bis zu 4 Kohlenstoffatomen stehen; sodann für Phenoxy, Naphthoxy und Phenylalkoxy mit bis zu 4 Kohlenstoffatomen im Alkylteil stehen; weiterhin noch für Halogen, Cyano- und Hydroxy-Gruppen stehen, und schließlich für Alkoxycarbonyl- mit bis zu 4 und für Dialkylamino-Gruppen mit bis zu 3 Kohlenstoffatomen im Alkylteil stehen, wobei die beiden Alkylreste auch mit dem Aminostickstoffatom zu einem heterocyclischen Ring geschlossen sein können, und weiterhin zwei Reste

R und $R^1$ zusammen mit 2 Kohlenstoffatomen des Benzolringsystems einen carbocyclischen oder heterocyclischen 5- oder 6-Ring bilden können, und
$R^3$ für Wasserstoff und für Alkyl mit bis zu 4 Kohlenstoffen steht, wobei letzteres durch Halogen und Methoxy-Gruppen substituiert sein kann, ferner für Allyl, Cyclopentyl und Cyclohexyl, für Benzyl, Chlorbenzyl und für Phenyläthyl steht,

dadurch gekennzeichnet, daß man o-Hydroxyacetophenone der allgemeinen Formel II

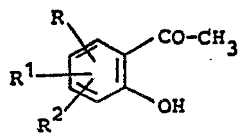
(II)

in welcher
R, $R^1$ und $R^2$ die oben angegebene Bedeutung haben, mit Glyoxylsäure-Derivaten der allgemeinen Formel III

$$OCH—COOZ \qquad (III)$$

in welcher
Z für ein- oder zweiwertige Kationen von Alkali- und Erdalkalimetallen, sowie für das Ammonium-, Mono-, Di- und Trialkylammonium-Kation stehen, wobei die Alkylreste jeweils bis zu 2 Kohlenstoffatome enthalten,

in Gegenwart von basischen Verbindungen im Temperaturbereich zwischen −30 und +150°C umsetzt und noch gegebenenfalls die dabei nach dem Ansäuern erhaltenen Carbonsäuren in an sich bekannter Weise in die entsprechenden Ester überführt.

2. Verfahren nach Anspruch 1, dadurch gekennzeichnet, daß man als basische Verbindungen sekundäre Amine verwendet.

3. Verfahren nach Anspruch 1, dadurch gekennzeichnet, daß man die Umsetzung im Temperaturbereich zwischen +20 und +80°C durchführt.

4. Chromon-Derivate der allgemeinen Formel Ia

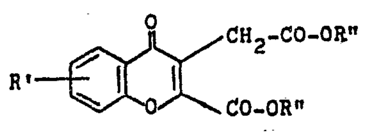
(Ia)

in welcher
R′ für Wasserstoff, Chlor oder Methoxy steht,

und

R″ für Wasserstoff oder Methyl steht.

5. Fungizide und die Entwicklung von Arthropoden hemmende Mittel, gekennzeichnet durch einen Gehalt an mindestens einem Chromon-Derivat gemäß Anspruch 4.

6. Verwendung von Chromon-Derivaten gemäß Anspruch 4 zur Bekämpfung von Pilzen und zur Entwicklungshemmung von Arthropoden.

**Claims**

1. Process for the preparation of chromone derivatives of the general formula I

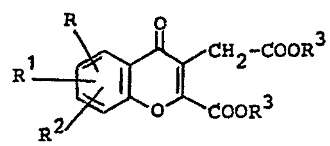
(I)

in which
R, $R^1$ and $R^2$ can be identical or different and represent hydrogen, and alkyl, cycloalkyl, alkenyl and cycloalkenyl with in each case up to 8 carbon atoms, the alkyl or cycloalkyl radicals optionally being able to be substituted by halogen, cyano, alkoxy, and alkoxycarbonyl groups with up to 4 carbon atoms; and also phenyl, naphthyl, phenylalkyl and naphthylalkyl with 1 to 4 carbon atoms in the

alkyl part, the named aromatic radicals being able to be substituted by halogen, cyano, and alkyl, alkoxy, alkoxycarbonyl and alkoxycarbonylalkyl groups with up to 4 carbon atoms and dialkylamino groups with a total of up to 6 carbon atoms; and also alkoxy, optionally substituted by halogen or cyano, with up to 4 carbon atoms; also phenoxy, naphthoxy and phenylalkoxy with up to 4 carbon atoms in the alkyl part, furthermore also halogen, cyano and hydroxy groups and finally alkoxycarbonyl groups with up to 4 carbon atoms in the alkyl part and dialkylamino groups with up to 3 carbon atoms in the alkyl part, the two alkyl radicals also being able to be cyclised, with the amino-nitrogen atom, to form a heterocyclic ring, and furthermore two radicals

R and $R^1$ together with 2 carbon atoms of the benzene ring system can form a carbocyclic or heterocyclic 5-membered or 6-membered ring and

$R^3$ represents hydrogen and alkyl with up to 4 carbon atoms, alkyl being able to be substituted by halogen and methoxy groups, also allyl, cyclopentyl and cyclohexyl, and benzyl, chlorobenzyl, and phenylethyl, characterised in that o-hydroxyacetophenones of the general formula II

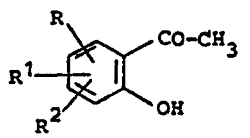

(II)

in which

R, $R^1$ and $R^2$ have the abovementioned meaning, are reacted with glyoxylic acid derivatives of the general formula III

$$OCH—COOZ \quad (III)$$

in which

Z represents monovalent or divalent cations of alkali metals and alkaline earth metals, as well as the ammonium cation and the monoalkylammonium, dialkylammonium and trialkylammonium cation, the alkyl radicals in each case containing up to 2 carbon atoms,

in the presence of basic compounds in the temperature range of between −30 and +150°C and in addition, if desired, the carboxylic acids obtained after acidification are converted in a manner which is in itself known into the corresponding esters.

2. Process according to claim 1, characterised in that secondary amines are used as basic compounds.

3. Process according to claim 1, characterised in that the reaction is carred out in the temperature range of between +20 and +80°C.

4. Chromone derivatives of the general formula Ia

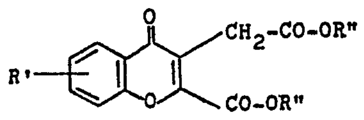

(Ia)

in which

R′ represents hydrogen, chlorine or methoxy,
and

R″ represents hydrogen or methyl.

5. Fungicidal agents and agents which inhibit the development of arthropods, characterised in that they contain at least one chromone derivative according to claim 4.

6. Use of chromone derivatives according to claim 4 for combating fungi and for inhibiting the development of arthropods.

**Revendications**

1. Procédé pour la préparation de dérivés de chromone de formule générale

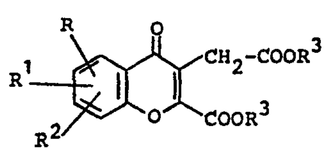

(I)

dans laquelle

R, $R^1$ et $R^2$ peuvent être identiques ou différents et représentent l'hydrogène ou un groupe alkyle, cycloalkyle, alcényle, cycloalcényle, chacun jusqu'en $C_8$, les restes alkyle ou cycloalkyle pouvant

être éventuellement substitués par des halogènes ou des groupes cyano, alkoxy ou alkoxycarbonyle jusqu'en $C_4$; en outre, un groupe phényle, naphtyle, phénylalkyle et naphtylalkyle, le reste alkyle ayant 1 à 4 atomes de carbone; les restes aromatiques mentionés pouvant être substitués par des halogènes ou des groupes cyano, alkyle, alkoxy, alcoxycarbonyle et alkoxycarbonylalkyle jusqu'en $C_4$ et des groupes dialkylamino ayant au total jusqu'à 6 atomes de carbone; en outre, des groupes alkoxy jusqu'en $C_4$ éventuellement substitués par des halogènes ou des groupes cyano; ensuite des groupes phénoxy, naphtoxy et phényl-alkoxy jusqu'en $C_4$; encore des halogènes, des groupes cyano ou hydroxy; et enfin des groupes alkoxycarbonyle jusqu'en $C_4$ et di(alkyl jusqu'en $C_3$)amino, les deux restes alkyle pouvant aussi former avec l'atome d'azote du groupe amino un noyau hétérocyclique; et, en outre, deux restes R et $R^1$ pouvant former avec deux atomes de carbone du noyau benzénique un noyau carbocyclique ou hétérocyclique à 5 ou 6 chaînons, et $R^3$ représente l'hydrogène ou un groupe alkyle jusqu-en $C_4$, ce dernier pouvant être substitué par des halogènes ou des groupes méthoxy, en outre un groupe allyle, cyclopentyle, cyclohexyle, benzyle, chlorobenzyle ou phényléthyle, caractérisé en ce que l'on fait réagir des o-hydroxyacétophénones de formule générale

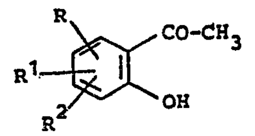

(II)

dans laquelle

R, $R^1$ et $R^2$ ont la signification indiquée ci-dessus, avec des dérivés d'acide glyoxylique de formule générale

$$OCH—COOZ \qquad (III)$$

dans laquelle

Z représente des cations mono- ou divalents de métaux alcalins et alcalino-terreux, ainsi que le cation ammonium, mono-, di- et trialkylammonium, les restes alkyle ayant chacun jusqu'à 2 atomes de carbone,

en présence de composés basiques dans un domaine de températures de —30 à +150°C, et l'on transforme encore éventuellement en esters correspondants, de manière connue, les acides carboxyliques ainsi obtenus après acidification.

2. Procédé selon la revendication 1, caractérisé en ce que l'on utilise comme composés basiques des amines secondaires.

3. Procédé selon la revendication 1, caractérisé en ce que l'on effectue la réaction dans un domaine de températures de +20 à +80°C.

4. Dérivés de chromone de formule générale (Ia)

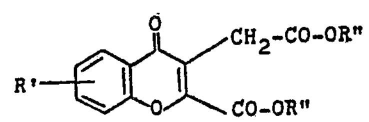

(Ia)

dans laquelle

R' représente l'hydrogène, le chlore ou un groupe méthoxy, et

R'' représente l'hydrogène ou un groupe méthyle.

5. Fongicides et agents inhibant le développement des arthropodes, caractérisés en ce qu'ils contiennent au moins un dérivé de chromone selon la revendication 4.

6. Utilisation des dérivés de chromone selon la revendication 4 pour la lutte contre les champignons et l'inhibition du développement des arthropodes.